(19) Europäisches Patentamt
European Patent Office
Office européen des brevets

(11) **EP 4 260 812 A1**

(12) **EUROPEAN PATENT APPLICATION**

(43) Date of publication:
**18.10.2023 Bulletin 2023/42**

(21) Application number: **22168339.4**

(22) Date of filing: **14.04.2022**

(51) International Patent Classification (IPC):
**A61B 8/06** (2006.01)     **A61B 5/029** (2006.01)
**A61B 8/08** (2006.01)

(52) Cooperative Patent Classification (CPC):
**A61B 8/5223; A61B 5/029; A61B 8/06;
A61B 8/0883**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **LAU, Kevin Daniel Seng Hung
Eindhoven (NL)**

• **MANZARI, Sabina
Eindhoven (NL)**
• **JOSHI, Rohan
Eindhoven (NL)**
• **BINGLEY, Peter
Eindhoven (NL)**
• **PALANISAMY, Krishnamoorthy
Eindhoven (NL)**

(74) Representative: **Philips Intellectual Property &
Standards
High Tech Campus 52
5656 AG Eindhoven (NL)**

(54) **DETERMINING A MEASUREMENT OF A HAEMODYNAMIC CHARACTERISTIC OF A SUBJECT**

(57)     According to an aspect, there is provided a computer-implemented method of determining a measurement of a haemodynamic characteristic of a subject. The method comprises obtaining (1001) an ultrasound waveform that comprises ultrasound data relating to a blood vessel of the subject. The ultrasound waveform relates to a time period that includes a plurality of heartbeat cycles of the subject. The method continues with processing (1003) the ultrasound waveform to detect a plurality of events in the ultrasound waveform, wherein the event is representative of a part of the heart beat cycle; calculating (1005) respective values of a haemodynamic characteristic from the detected events; processing (1007) the calculated values of the haemodynamic characteristic to correct for irregularities in the ultrasound waveform and/or in the values of the haemodynamic characteristic, and to generate corrected values of the haemodynamic characteristic; and outputting (1009), via a display unit, the corrected values of the haemodynamic characteristic.

Fig. 10

EP 4 260 812 A1

**Description**

FIELD OF THE INVENTION

This disclosure relates to techniques for determining a measurement of a haemodynamic characteristic of a subject, and in particular to techniques for determining such measurements from an ultrasound waveform.

BACKGROUND OF THE INVENTION

In number of situations, the health status of an individual or subject needs to be monitored, which is often done by the measurement of vital signs, such as heart rate, blood pressure, respiration rate, cardiac output, pressure and stroke volume variation, etc. Such vital signs (or haemodynamic characteristics) can be derived from a number of signals, such as electrocardiogram (ECG) and/or ultrasound (US) signals.

In situations in which an ultrasound device is used as the only monitoring device on a subject, it is required to derive the haemodynamic characteristics from just an ultrasound waveform derived from ultrasound image data. The ultrasound waveform can represent blood velocity and/or artery diameter. Such situations can arise where the subject has a wearable ultrasound device which operates independently and does not have (additional) input from other monitoring systems (such as an ECG waveform from an ECG system). The reliable calculation of vital signs is important for the clinical adoption of wearable ultrasound devices.

Values of haemodynamic characteristics are typically derived from landmarks and numerical values (referred to as waveform features) determined from full signal waveforms. For example, the heart rate is a waveform feature and can be calculated from landmarks that define the duration of a heartbeat, such as the so-called R-peak of an ECG waveform.

The calculated heart rate (HR) is also required to calculate other haemodynamic characteristics/waveform features such as cardiac output (CO), which is defined as the heart rate multiplied with the stroke volume (SV):

$$CO = HR \times SV \qquad (1)$$

SV is a waveform feature defined as the total volume of blood over a given time period. This can be computed by integrating the area under the blood velocity curve, converting to mean velocity (assuming the shape of the velocity profile), and then multiplying by the cross-sectional area of the blood vessel. These are all features of the ultrasound waveform and can also be impacted by physiological variations and US-related noise. Since multiple waveform features are multiplied together to compute a haemodynamic characteristic/waveform feature, such as CO, this can result in erroneous values of CO, due to the cumulative impact of irregularities or errors in the different waveform features used to derive CO.

Other haemodynamic characteristics such as stroke volume variation (SVV) depend upon the maximum and minimum value of the stroke volume over a fixed period ($SV_{\text{Max}}$ and $SV_{\text{Min}}$ respectively):

$$SVV = \frac{SV_{\text{Max}} - SV_{\text{Min}}}{0.5(SV_{\text{Max}} + SV_{\text{Min}})} \qquad (2)$$

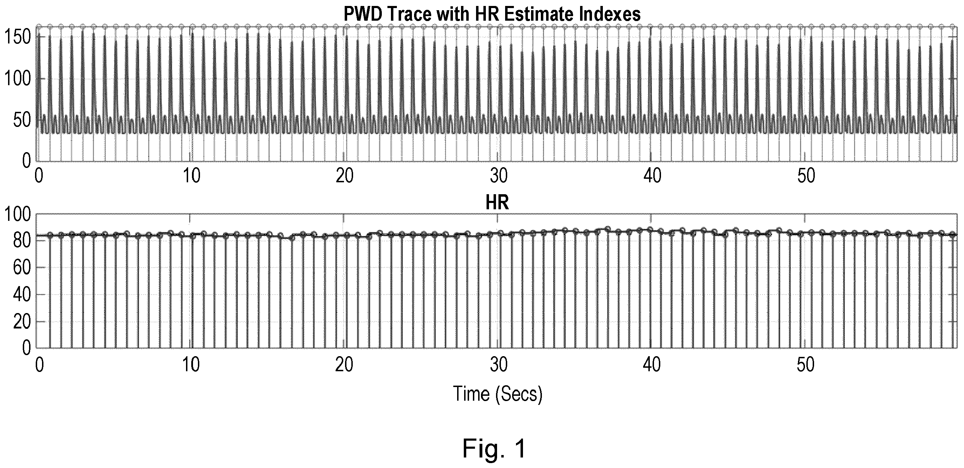

When using only ultrasound waveforms, for example ultrasound measurements of blood velocity, vital signs such as the heart rate can also be measured, as illustrated in Fig. 1, which shows a blood velocity waveform in which suitable landmarks/events are used to determine the duration of a heartbeat. The top graph in Fig. 1 is an ultrasound velocity waveform (a pulsed wave Doppler (PWD) trace) obtained from a subject that has a constant heart rate in the measurement window and shows the extracted ultrasound waveform and landmark detection, and the bottom graph in Fig. 1 shows the calculation of heart rate using the time interval between events/landmarks. In the top graph, the peak velocity value per beat, as indicated by the circles, are used as the landmark for the heartbeat duration and the heart rate.

The example blood velocity waveform in Fig. 1 shows a constant heart rate with no waveform irregularities. However, waveform irregularities can occur, for example due to physiological variations and/or image artefacts due to relative movement between the subject and the ultrasound probe used to obtain the ultrasound waveform or electromagnetic interference from external sources such as electrosurgical devices.

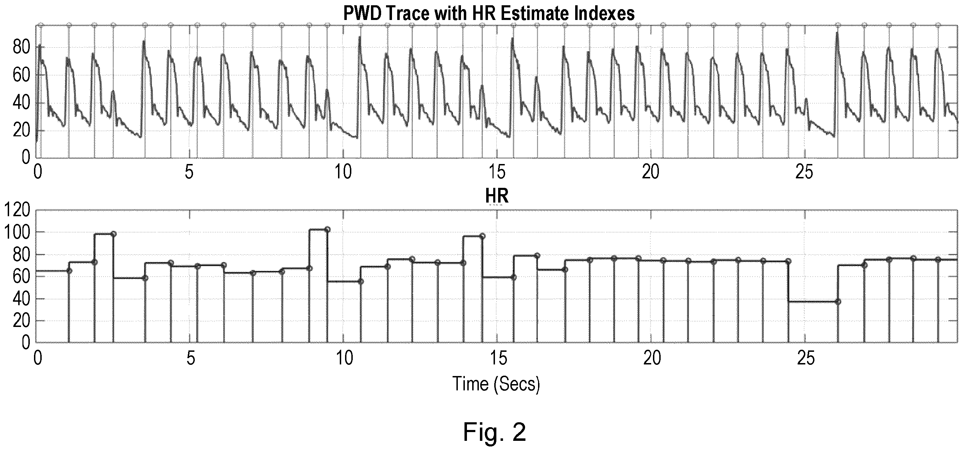

Fig. 2 illustrates an example waveform in which irregularities due to physiological variations are present (i.e. the subject's heart rate is irregular) and the peaks associated with those irregularities in the blood velocity measurement are generally not detected (although in Fig. 2 some of the extra-systolic peaks have been detected), which results in irregularities in the heart rate determined from the ultrasound signal. The top graph in Fig. 2 shows an ultrasound velocity waveform obtained from a subject. However, in contrast to the velocity waveform in Fig. 1, the velocity waveform in Fig. 2 has irregularities due to extra-systolic beats. The top graph in Fig. 2 also shows the extracted ultrasound waveform and landmark detection, and the bottom graph in Fig. 2 shows the calculation of heart rate using the time interval between events/landmarks. In this example the extra-systolic beats in the waveform that have not been detected and have not been used to determine the heart rate, and an irregular heart rate is observed.

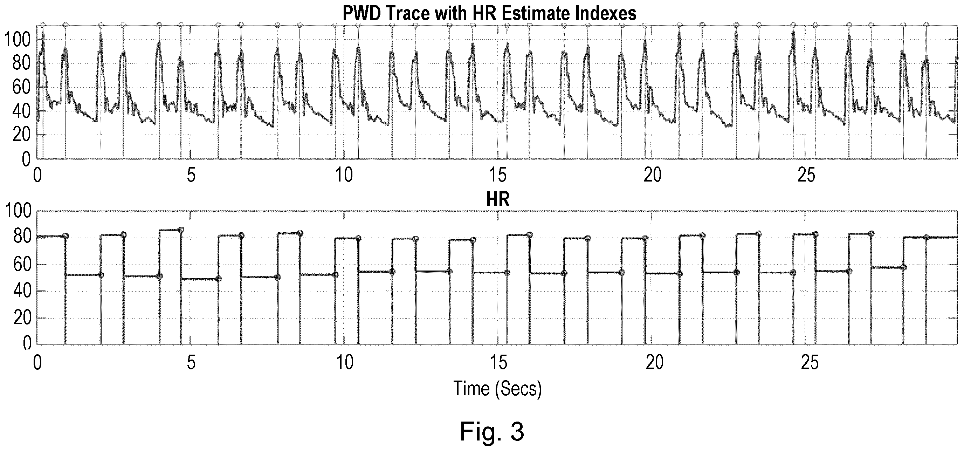

Fig. 3 illustrates an example waveform in which the subject has an irregular heartbeat, but all peaks are detected in the signal. This results in a heart rate measurement that oscillates. The top graph in Fig. 3 shows an ultrasound velocity waveform obtained from a subject. The top graph in Fig. 3 shows the extracted ultrasound waveform and landmark detection, and the bottom graph in Fig. 3 shows the calculation of heart rate using the time interval between events/landmarks.

Both Fig. 2 and Fig. 3 show examples where the clinical output should be processed or evaluated before being provided to clinicians, as otherwise false positives could occur. The occurrence of such extra-systolic beats as shown in Figs. 2 and 3 results in beat-by-beat variation in heart rate, which in many cases is not sustained and is therefore not life threatening. Displaying such variation on a patient monitor could therefore be misinterpreted by the subject or by their healthcare team. Other methods of heart rate estimation, such as those based on ECG signals, perform filtering to address such variations.

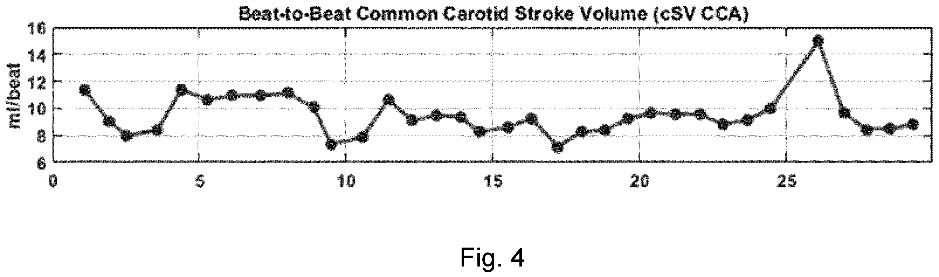

Furthermore, other haemodynamic characteristics such as stroke volume are calculated beat-to-beat using the same ultrasound waveform events used for heartbeat detection, to provide a sequence of stroke volume values that are used to calculate the SVV. In the presence of irregular beats, the calculation of the SVV can be affected by an abnormally small value of $SV_{Min}$ or large $SV_{Max}$ value, as illustrated in Fig. 4. Fig. 4 shows the calculated beat-to-beat common carotid stroke volume (cSV) as calculated from the example waveform in Fig. 2. In particular, in Fig. 4 the extra-systolic beat at around 26 seconds results in a significantly higher stroke volume than the other beats, resulting in a significantly increased SVV than would otherwise be considered.

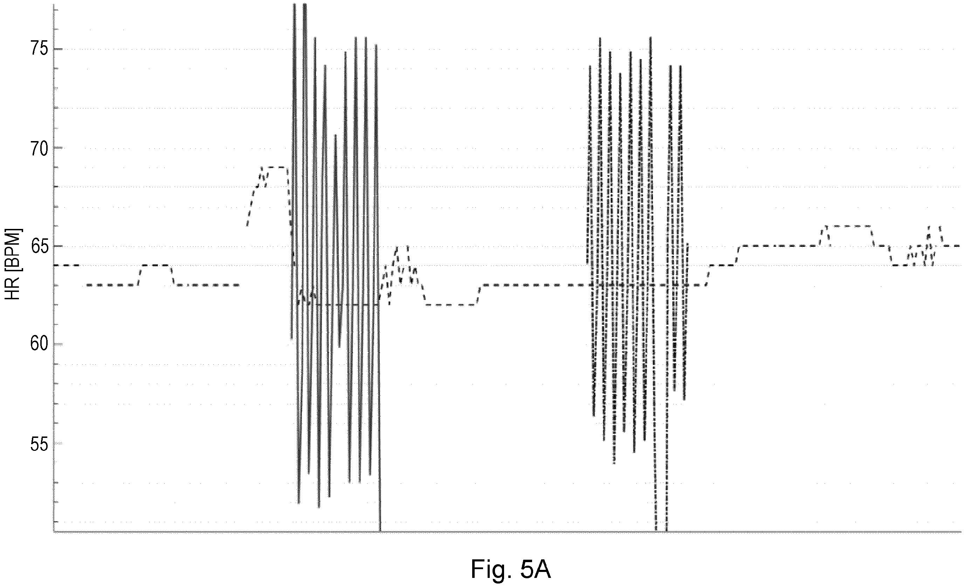

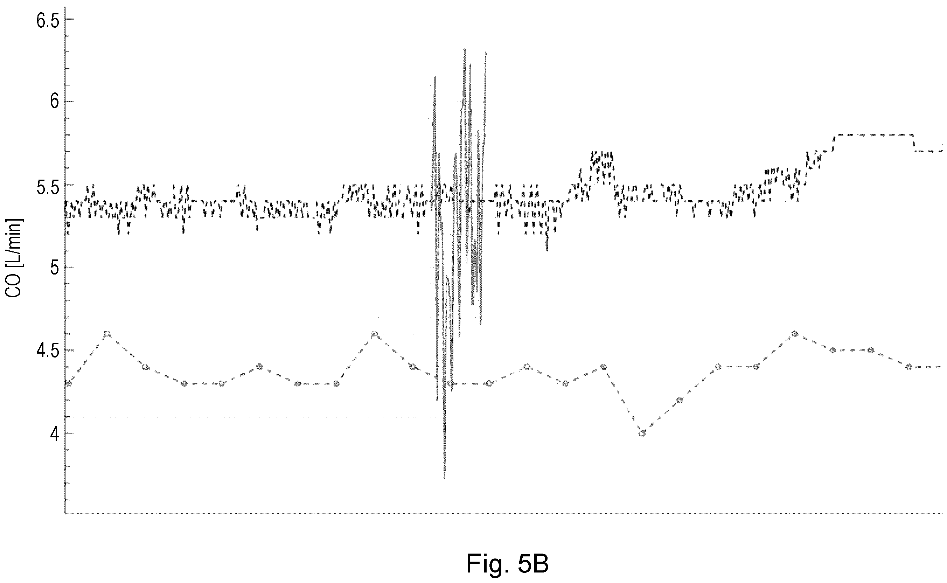

As shown in Fig. 5, the irregular heart rate variations in the ultrasound waveform result in both large variations in the heart rate and in the cardiac output as compared to other reference methods (e.g. heart rate from ECG and cardiac output from thermodilution). As noted, for an ultrasound probe, one reason for waveform irregularities to occur is due to relative movement between the subject and the ultrasound probe used to obtain the ultrasound waveform (where the ultrasound probe can typically be positioned on or near the neck of the subject). This is not such a problem for ECG signals since ECG electrodes can be attached to the skin and placed on the chest of the subject. The graph in Fig. 5A shows a heart rate waveform derived from an ECG signal (the dashed line) and a heart rate waveform derived from an ultrasound waveform obtained for the carotid (solid line) and brachial arteries (dot-dashed line). The graph in Fig. 5B shows an estimated cardiac output waveform (dashed line) derived from an ultrasound waveform (solid line) compared to reference measurements of CO via thermodilution methods (the top line). It is noted that cardiac output is a measure of the global output of the heart and measuring at the carotid or brachial artery means that the cardiac output is estimated from these local measurements. Fig. 5A shows that there are outliers in the ultrasound-derived heart rate when compared to the reference heart rate (ECG-derived heart rate). If this ultrasound-derived heart rate is used without correcting for the irregularities, then the resulting heart rate can fluctuate significantly. Similar fluctuations can be seen in the calculated CO in Fig. 5B.

Thus, there is a need for improvements in the calculation of values of haemodynamic characteristics from ultrasound signals.

SUMMARY OF THE INVENTION

According to a first specific aspect, there is provided a computer-implemented method of determining a measurement of a haemodynamic characteristic of a subject. The method comprises obtaining an ultrasound waveform that comprises ultrasound data relating to a blood vessel of the subject, wherein the ultrasound waveform relates to a time period that includes a plurality of heart beat cycles of the subject; processing the ultrasound waveform to detect a plurality of events in the ultrasound waveform, wherein the event is representative of a part of the heart beat cycle; calculating respective values of a haemodynamic characteristic from the detected events; processing the calculated values of the haemodynamic characteristic to correct for irregularities in the ultrasound waveform and/or in the values of the haemodynamic characteristic, and to generate corrected values of the haemodynamic characteristic; and outputting, via a display unit, the corrected values of the haemodynamic characteristic.

According to a second aspect, there is provided a computer program product comprising a computer readable medium having computer readable code embodied therein, the computer readable code being configured such that, on execution by a suitable computer or processor, the computer or processor is caused to perform the method according to the first aspect or any embodiment thereof.

According to a third aspect, there is provided an apparatus configured to determine a measurement of a haemodynamic characteristic of a subject. The apparatus comprises a processing unit and a display unit, and the processing unit is configured to: receive an ultrasound waveform that comprises ultrasound data relating to a blood vessel of the subject, wherein the ultrasound waveform relates to a time period that includes a plurality of heart beat cycles of the subject; process the ultrasound waveform to detect a plurality of events in the ultrasound waveform, wherein the event is representative of a part of the heart beat cycle; calculate respective values of a haemodynamic characteristic from the detected events; process the calculated values of the haemodynamic characteristic to correct for irregularities in the ultrasound waveform and/or in the values of the haemodynamic characteristic, and to generate corrected values of the haemodynamic characteristic; and output, via the display unit, the corrected values of the haemodynamic characteristic.

These and other aspects will be apparent from and elucidated with reference to the embodiment(s) described hereinafter.

It will be appreciated by those skilled in the art that two or more of the above-mentioned options, implementations, and/or aspects of the invention may be combined in any way deemed useful.

BRIEF DESCRIPTION OF THE DRAWINGS

Exemplary embodiments will now be described, by way of example only, with reference to the following drawings, in which:

Fig. 1 shows a blood velocity waveform in which suitable landmarks/events are used to determine the duration of a heartbeat and therefore heart rate;

Fig. 2 shows the heart rate determined from an exemplary waveform in which the subject has an irregular heartbeat and many irregularities in the waveform are ignored;

Fig. 3 shows the heart rate determined from an example waveform in which the subject has an irregular heartbeat, and all peaks are detected in the waveform.

Fig. 4 illustrates the effect of irregular heartbeats on the calculation of stroke volume (SV);

Figs. 5A and 5B provide a comparison between ultrasound and ECG-based methods for determining heart rate and between ultrasound and thermodilution for determining cardiac output;

Fig. 6 illustrates an apparatus according to various embodiments;

Fig. 7 shows two examples of ultrasound signals that can be processed according various embodiments;

Fig. 8 illustrates consistent event detection in an ultrasound signal;

Fig. 9 illustrates the correction of irregular waveforms in an ultrasound signal; and

Fig. 10 illustrates a method of determining a measurement of a haemodynamic characteristic of a subject according to various embodiments.

DETAILED DESCRIPTION OF EMBODIMENTS

As noted above, there is a need for improvements in the calculation of values of haemodynamic characteristics from ultrasound waveforms since irregularities in the ultrasound waveform can lead to incorrect values of the haemodynamic characteristics. One particular haemodynamic characteristic that can be calculated from the ultrasound waveform is the heart rate of the subject, although other haemodynamic characteristics can be calculated, such as cardiac output, stroke volume, stroke volume variation, etc.

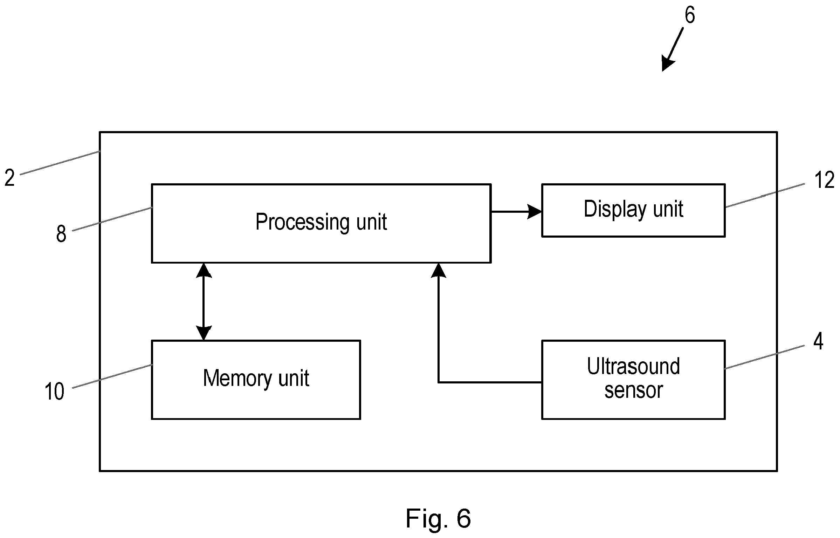

Fig. 6 illustrates an apparatus 2 according to various embodiments of the present disclosure. The apparatus 2 is for determining a measurement of a haemodynamic characteristic of a subject from an ultrasound waveform. The ultrasound waveform is obtained using an ultrasound sensor 4, which is to be placed on or near a part of the body of a subject and used to obtain an ultrasound waveform comprising ultrasound data relating to a blood vessel of the subject. An ultrasound sensor 4 according to the present disclosure can take or be coupled to a plurality of form factors, such as, for example, an external (handheld) ultrasound probe, an ultrasound patch, an internal ultrasound probe. The ultrasound waveform will be obtained over a period of time that is long enough to include measurements of multiple heartbeat cycles of the subject. The terms "heartbeat cycle" and "heartbeat" are used interchangeably herein.

In some embodiments, the ultrasound sensor 4 can be configured to be used at or near a neck of a subject and used to measure blood flow in an artery or vein in the neck, such as the carotid artery. In some embodiments, the ultrasound data obtained by the ultrasound sensor 4 relating to the blood vessel of the subject is measurements of the blood velocity (or changes in the blood velocity) of the blood in the blood vessel and measurements of the diameter (or changes in the diameter) of the blood vessel over time. In some embodiments, the ultrasound data obtained by the ultrasound sensor 4 relating to the blood vessel of the subject is one of the blood velocity and diameter.

In the embodiment shown in Fig. 6, the ultrasound sensor 4 is part of the apparatus 2, i.e. part of the apparatus 2 that processes the ultrasound waveform to determine values of one or more haemodynamic characteristics of the subject. For example, the apparatus 2 can be in the form of a portable or wearable device that can be carried or worn by a subject and used to continuously or regularly determine values of haemodynamic characteristics for the subject using ultrasound data obtained by the ultrasound sensor 4. Alternatively, the apparatus 2 can be part of a measurement system 6 that comprises the apparatus 2 and a separate ultrasound sensor 4. In these embodiments, the apparatus 2 can be in the form of a patient monitor.

The apparatus 2 includes a processing unit 8 that controls the operation of the apparatus 2 and that can be configured to execute or perform the methods described herein. The processing unit 8 can be implemented in numerous ways, with software and/or hardware, to perform the various functions described herein. The processing unit 8 may comprise one or more microprocessors or digital signal processors (DSPs) that may be programmed using software or computer program code to perform the required functions and/or to control components of the processing unit 8 to effect the required functions. The processing unit 8 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 8 is connected to a memory unit 10 that can store data, information and/or signals for use by the processing unit 8 in controlling the operation of the apparatus 2 and/or in executing or performing the methods described herein. In some implementations the memory unit 10 stores computer-readable code that can be executed by the processing unit 8 so that the processing unit 8 performs one or more functions, including the methods described herein. In particular embodiments, the program code can be in the form of an application for a smartwatch, smartphone, tablet, laptop or computer. The memory unit 10 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit 10 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 2 also comprises a display unit 12, that enables the apparatus 2 to output information or data to a user of the apparatus 2 (e.g. to the subject themselves, or to a member of a healthcare team for the subject). The information or data output by the display unit 12 can include the ultrasound signal obtained by the ultrasound sensor 4, and/or values of one or more haemodynamic characteristics obtained from the ultrasound waveform.

Although not shown in Fig. 6, the apparatus 2 can include one or more further user interface components that enable a user of apparatus 2 to input information, data and/or commands into the apparatus 2. The user interface components can be any of a keyboard, keypad, one or more buttons, switches or dials, a mouse, a track pad, a touchscreen for the display unit 12, a stylus, a camera, a microphone, etc.

The apparatus 2 can be any type of electronic device or computing device. In some implementations, as noted above, the apparatus 2 can be an apparatus that is carried or worn by the subject. For example, the apparatus 2 can be, or be part of, a laptop, a tablet, a smartphone, a smartwatch, etc. In other implementations, the apparatus 2 is an apparatus that is present or used in the home or care environment of the subject/user. For example, the apparatus 2 can be, or be part of, a laptop, a tablet, a smartphone, a smartwatch, a computer, a bedside monitor or a patient monitor. In other implementations, the apparatus 2 is an apparatus that is remote from the subject, and remote from the home or care environment of the subject. For example, the apparatus 2 can be a server, for example a server in a data center (also referred to as being 'in the cloud').

It will be appreciated that a practical implementation of an apparatus 2 may include additional components to those shown in Fig. 6. For example, the apparatus 2 may also include a power supply, such as a battery, or components for enabling the apparatus 2 to be connected to a mains power supply.

In particular embodiments, the apparatus 2 (and specifically the processing unit 8) can be configured to operate as follows. The apparatus 2/processing unit 8 can receive a blood velocity waveform and/or blood vessel diameter waveform from the ultrasound sensor 4, and robustly detect events in the ultrasound waveform(s). The processing unit 8 performs a consistent calculation of vital signs/haemodynamic characteristics from the detected events. In some embodiments, the processing unit 8 performs or implements a modification of irregular parts of an ultrasound waveform for haemodynamic characteristic calculation. In some embodiments, based on the determined haemodynamic characteristic values, the apparatus processing unit 8 can automatically detect life threatening irregularities in the haemodynamic characteristic values.

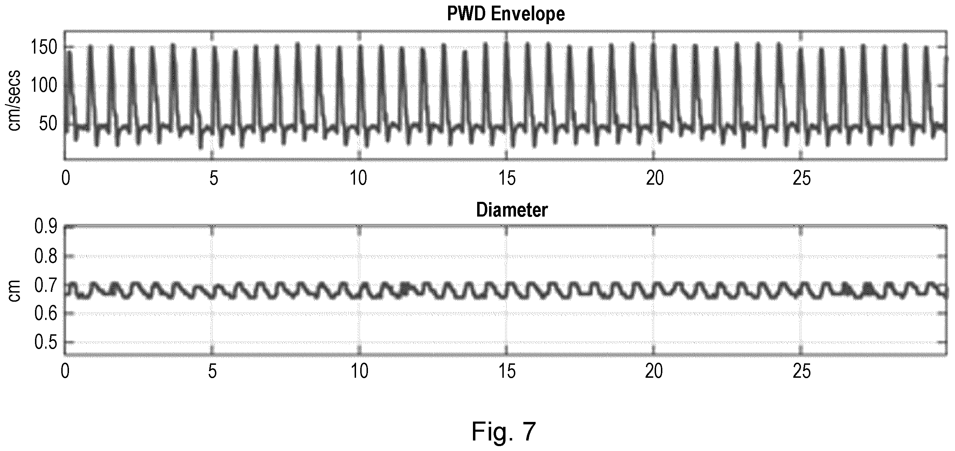

Fig. 7 shows an example of the ultrasound signals (a pulsed wave Doppler (PWD) envelope) that can be obtained and processed according to the techniques described herein. The top graph in Fig. 7 shows an ultrasound waveform (PWD envelope) comprising ultrasound data in the form of blood velocity measurements. The bottom graph in Fig. 7 shows an ultrasound waveform comprising ultrasound data in the form of blood vessel diameter measurements. In some embodiments, one or more haemodynamic characteristics are determined by processing both ultrasound data in the form of blood velocity measurements and ultrasound data in the form of blood vessel diameter measurements.

To provide a robust detection of events in the ultrasound waveform, different features of the specific ultrasound waveform can be detected, and the duration of the heartbeat of the subject can be calculated. In Figs. 1, 2 and 3, the event detected is the maximum value of the blood velocity in each heartbeat, and this detected event is used to define each beat. However, alternative approaches also exist that make use of different events, for example the foot (minimum) of the waveform typically found at the end of the diastole phase/beginning of the systole phase can also be used to determine the heartbeat duration, or the part of the waveform corresponding to the dicrotic notch (which denotes the end of the systole phase and the beginning of the diastole phase) can allow the systolic duration to be determined. In the techniques described herein, a simple event detection algorithm is used to detect all events (e.g. all peaks/maxima/troughs/minima/maximum or minimum derivatives of the waveform values, etc.). The event detection algorithm does not discriminate whether any particular event is relevant/reliable or not. Instead, the techniques described herein provide that there is processing of the detected events to determine a reliable clinical value.

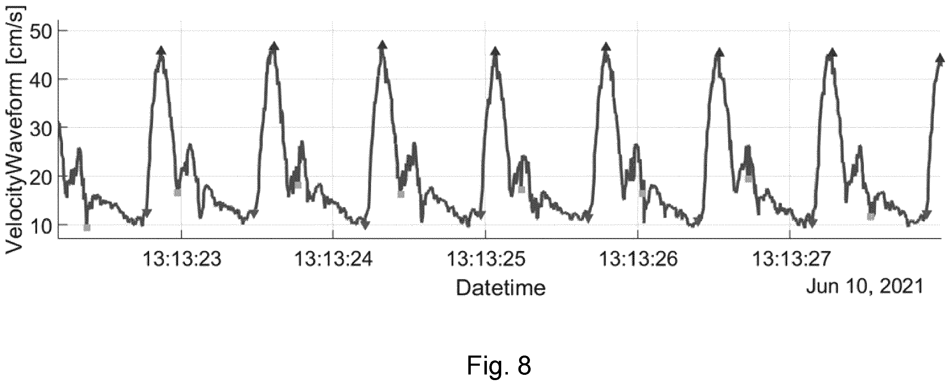

Fig. 8 illustrates three different types of events that can be detected in a blood velocity waveform. A first type of event, marked with an upright triangle, corresponds to the peaks in the waveform. A second type of event, marked with an inverted triangle, corresponds to the foot of the peaks in the waveform. A third type of event, marked with a square, corresponds to the end of the systole phase.

Using the detected events from the blood velocity waveform (or blood vessel diameter waveform), a haemodynamic characteristic such as the heart rate can be computed from the heartbeat duration, as shown in Figs. 1-3. Both types of waveform (velocity and diameter) are used to calculate the stroke volume via a transfer function, and then the calculated heart rate and stroke volume can be used to calculate the cardiac output (CO) via a transfer function. The duration of the heartbeat can be determined as the time elapsed between two consecutive detected events. Alternatively, the duration of the heartbeat can be determined from detected events that relate to a plurality of heart beats. In this case, a mathematical function can be used to determine the duration of the heartbeat from the detected events. The mathematical function can be based on any suitable principle, such as averaging (mean, mode, median), or a statistical measure (e.g. standard deviation). Algorithms for detecting these types of landmarks/events already exist, and those skilled in the art will be aware of suitable algorithms that can be used.

However, as noted above, irregularities in the blood velocity waveform or vessel diameter waveform can result in large variations in estimated heart rate and stroke volume. Therefore, embodiments of the techniques described herein provide one or more actions are taken to correct for irregularities in the ultrasound waveform. These actions can comprise processing the values of the haemodynamic characteristic to correct for irregularities in the haemodynamic characteristic values caused by the irregularities in the ultrasound waveform. These actions can also or alternatively comprise processing the detected events to correct irregular events and/or missing events in the ultrasound waveform. These actions can also or alternatively comprise processing the ultrasound waveform itself to correct irregularities in the ultrasound waveform.

Embodiments in which the values of the haemodynamic characteristic are processed to correct for irregularities in the haemodynamic characteristic values can comprise determining a beat-to-beat variation in the haemodynamic characteristic, and then smoothing the beat-to-beat variation. The smoothing of the beat-to-beat variation can be performed using any suitable technique, such as linear interpolation, cubic interpolation, moving average, median, etc. Alternatively or in addition, correcting for irregularities in the haemodynamic characteristic values can comprise computing an average (e.g. mean, mode or median) of the haemodynamic characteristic values over a particular time window. Similarly, this approach can be also used for other computed vital signs such as the cardiac output.

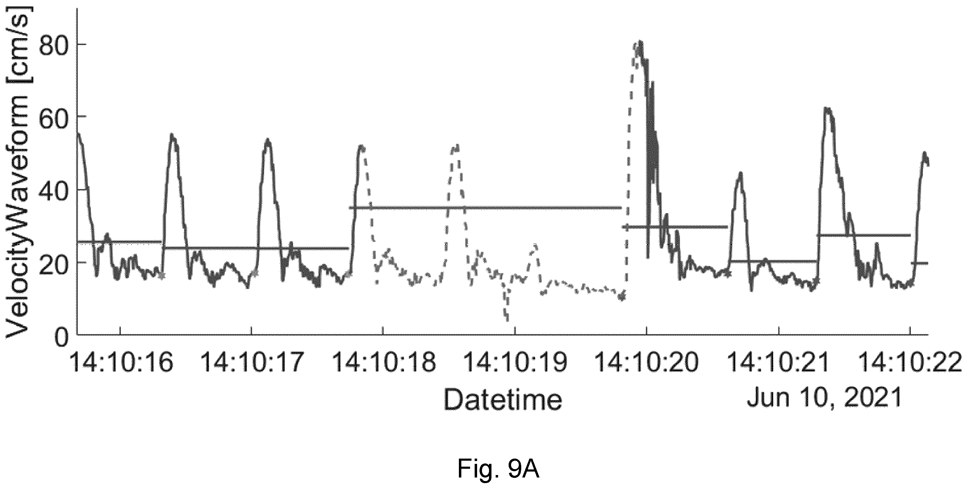

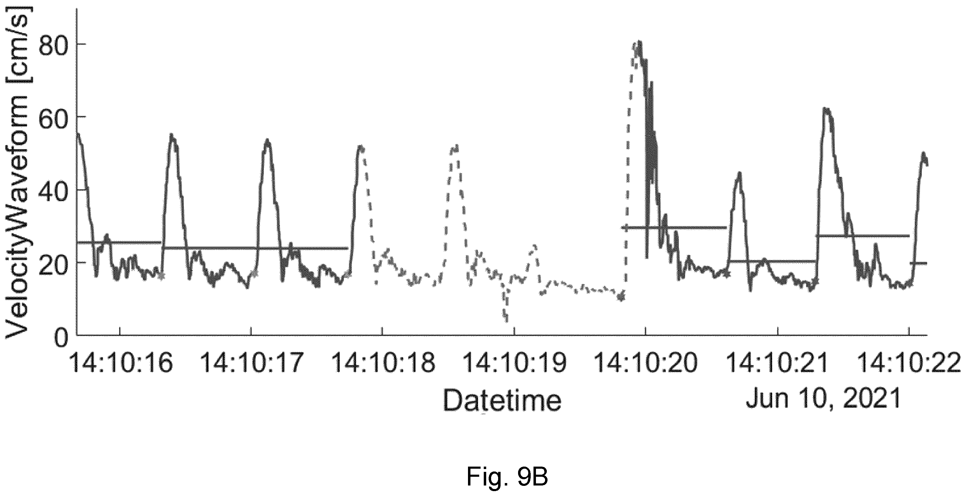

As illustrated in Fig. 4, irregular heartbeats result in higher stroke volume values that can impact the calculation of SVV. Two approaches are proposed to solve this issue. The first is to remove any values of the stroke volume (or other haemodynamic characteristic) that are significantly higher from the calculation of the SVV. An illustration of removing an irregular beat for SVV calculation is provided with reference to Figs. 9A and 9B. Fig. 9A shows a velocity waveform having an irregular feature caused by irregular beats. The horizontal lines superimposed on the waveform indicate the beat-by-beat stroke volume value derived from that part of the waveform. Firstly, an irregular beat is detected (the dashed signal region in the velocity waveform in Fig. 9A). Next, that part of the ultrasound waveform (i.e. associated with the irregular stroke volume value) and the irregular stroke volume value is removed from the extracted values. This is shown in Fig. 9B where the stroke volume value(s) for the dashed signal region are removed. Then, the corrected beat-to-beat variation in stroke volume is calculated based on the remaining parts of the waveform having valid stroke volume values.

The criteria used to determine whether to remove an irregular stroke volume value (or other haemodynamic characteristic value) can use any suitable method. For example it can be based on a deviation of the stroke volume value from a mean stroke volume, or as a percentage difference from the stroke volume associated with the previous heartbeat, or it can be based on features of the ultrasound waveform, etc. Regarding features of the waveform, these can be characteristics of the velocity waveform, for instance the peak systolic velocity which is likely to be significantly different in an irregular beat than in a regular beat. Once the irregular stroke volume value (or other haemodynamic characteristic value) is removed, the SVV (or other haemodynamic characteristic value) can be calculated using the remaining values.

The above approach has the added benefit that once the irregular beat is removed, the modified waveform can also be used to recompute the heart rate.

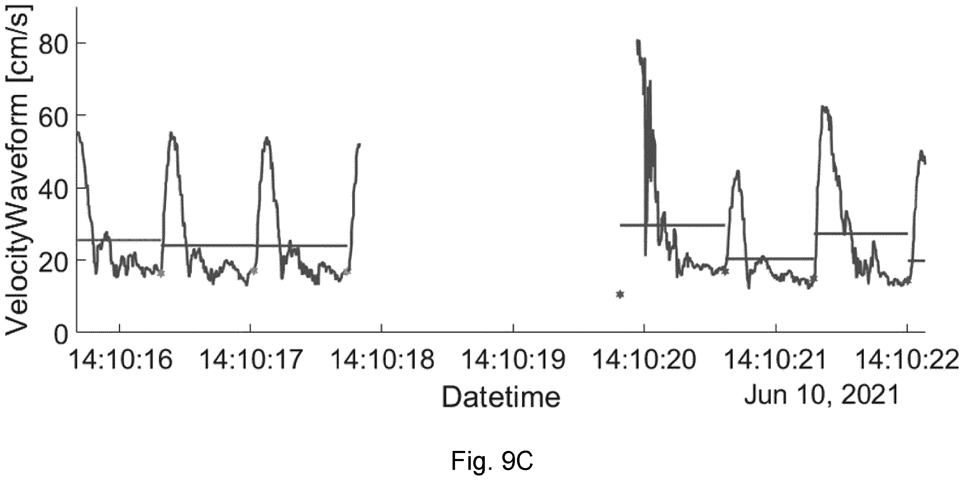

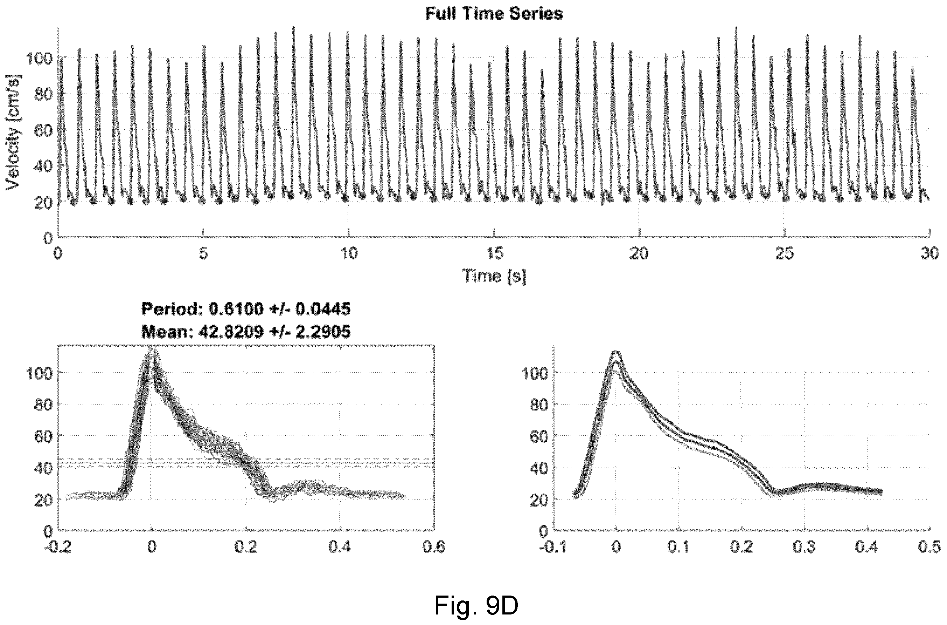

The second approach is an extension of the first approach in which the part of the waveform corresponding to the removed (irregular) beat is replaced with a 'corrected' beat/waveform, as illustrated in Figs. 9C and 9D. In this approach, the (irregular) beat is removed - as shown in Fig. 9C and replaced with a statistically similar waveform based on (e.g. a subject-specific) one or more waveforms to create an ensemble average of the subject's waveform, which is illustrated in Fig 9D. In Fig. 9D, a subject-specific template waveform (as shown in the bottom right of Fig. 9D) is created from an ensemble average of all or a subset of the preceding valid beats (shown in the top part of Fig. 9D). In the waveform in the top part of Fig. 9D, the individual waveforms are separated by using selected landmarks, e.g. the peak systolic velocity (as shown in the bottom left part of Fig. 9D). Alternatively, a template waveform can be created or obtained from a populational database, based on demographics such as age, sex, body mass index (BMI), carotid anatomy, etc. Once the irregular waveform beat has been replaced with the statistically similar waveform or template waveform, the stroke volume (or other haemodynamic characteristic) is recalculated.

The above embodiments describe corrections for the occurrence of occasional, non-life-threatening, waveform irregularities. Such irregularities may be motion artefacts resulting from relative movement of the ultrasound sensor 4 and the subject, poor contact between the ultrasound sensor 4 and the subject, or electronic noise. However, persistent irregularities could be a sign of a life-threatening event, such as ventricular tachycardia, ventricular fibrillation, premature ventricular contraction, atrial fibrillation, extra-systolic beats, bleeding, etc. Therefore, embodiments provide that the number and/or duration of irregular beats can be monitored or tracked, and the number and/or duration of irregular beats can be used to detect life-threatening events or other events that require treatment or an intervention from a healthcare team. For example, the number and/or duration can be compared to one or more thresholds to determine if a life-threatening event has occurred. In some embodiments, the number and/or duration of the irregularities occurring within a particular time period can be compared to one or more thresholds. The threshold(s) may be static, e.g. set for the particular subject or for a population of subjects, or they may be dynamic, e.g. the threshold(s) may change based on trend information relating to an increasing or decreasing occurrence of irregular beats.

During episodes of consistent arrhythmias, the uncorrected haemodynamic characteristic(s) (e.g. heart rate, cardiac output, etc. derived from the ultrasound waveform) can be utilized to determine the severity/impact of the sustained arrhythmias, e.g. through the reduction in cardiac output. Furthermore, these uncorrected haemodynamic characteristic values can also be used to guide/assess effectiveness of drug therapy or emergency treatment such as cardiopulmonary resuscitation (CPR), by detecting a reduction in arrhythmic events. For example, drugs such as beta-blockers are used to reduce arrhythmias. Their efficacy is variable, and especially their efficacy in changing stroke volume is unknown. By measuring stroke volume associated with erroneous beats, the efficacy of drug therapy can be assessed. Similarly, for CPR, it is useful to know whether the CPR is ensuring blood flow to the brain. By assessing the SV associated with the uncorrected beats, this can be quantified, and feedback provided to the person administering CPR.

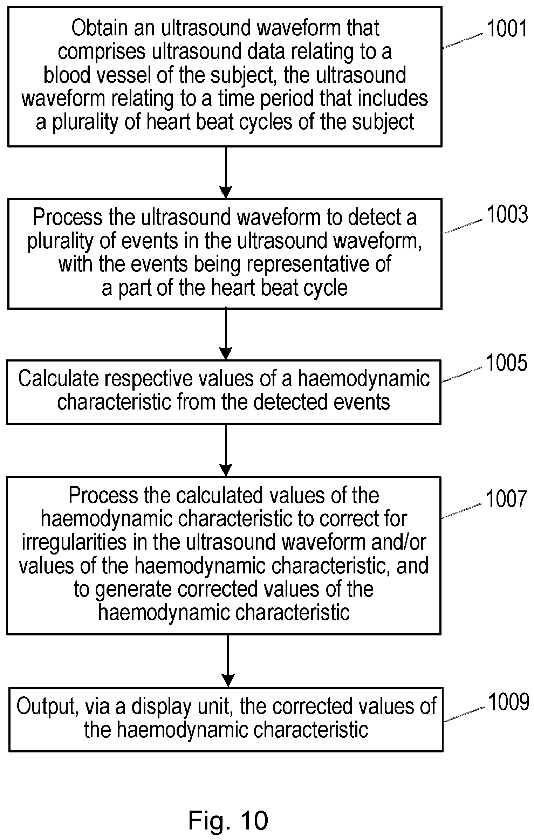

The flow chart in Fig. 10 illustrates an exemplary method according to the techniques described herein. One or more of the steps of the method can be performed by the processing unit 8 in the apparatus 2, in conjunction with any of the memory unit 10, ultrasound sensor 4 and display unit 12 as appropriate. The processing unit 8 may perform the one or more steps in response to executing computer program code, that can be stored on a computer readable medium, such as, for example, the memory unit 10.

In a first step, step 1001, an ultrasound waveform is obtained that comprises ultrasound data relating to a blood vessel of the subject. The ultrasound waveform relates to a time period that includes a plurality of heartbeat cycles of the subject. The ultrasound data may comprise measurements of blood velocity in a blood vessel, and/or measurements of blood vessel diameter of a blood vessel. In some embodiments, the ultrasound waveform comprises ultrasound data indicative of blood flow in a carotid artery of the subject.

In step 1003, the ultrasound waveform is processed to detect a plurality of events in the ultrasound waveform. Each event is representative of a part of the heartbeat cycle, for example a local maximum, a local minimum, or a local maximum/minimum of a derivative of the ultrasound waveform. Thus, step 1003 comprises identifying each occurrence of a particular event, e.g. a peak, foot of a peak, etc., in the ultrasound waveform.

In step 1005, respective values of a haemodynamic characteristic are calculated from the detected events. The haemodynamic characteristic may be heart rate or stroke volume.

In step 1007, the calculated values of the haemodynamic characteristic are processed to correct for irregularities in the ultrasound waveform and/or irregularities in the values of the haemodynamic characteristic to generate corrected values of the haemodynamic characteristic. The irregularities in the ultrasound signal may be a result of artefacts in ultrasound waveform.

In step 1009, the corrected values of the haemodynamic characteristic are output via a display unit.

In some embodiments, step 1007 comprises comparing the calculated values of the haemodynamic characteristic and smoothing a heartbeat-to-heart beat variation in the calculated values.

In some embodiments, step 1007 comprises, for a first calculated value of the haemodynamic characteristic, generating a corrected value for the first calculated value as an average of the calculated values of the haemodynamic characteristic in a time window that includes the first calculated value.

In some embodiments, step 1007 comprises identifying one or more calculated values that result from a respective irregular waveform in the ultrasound waveform; and discarding the one or more identified calculated values, wherein the corrected values of the haemodynamic characteristic are the non-discarded calculated values. Alternatively, step 1007 can comprise identifying one or more calculated values that result from a respective irregular waveform in the ultrasound waveform; and recalculating the values of the haemodynamic characteristic excluding parts of the ultrasound waveform associated with the one or more identified calculated values to calculate the corrected values. In another alternative, step 1007 can comprise identifying one or more calculated values that result from a respective irregular waveform in the ultrasound waveform; replacing the one or more irregular parts of the ultrasound waveform with corrected waveforms to generate a corrected ultrasound waveform; and repeating the steps of processing the ultrasound waveform on the corrected ultrasound waveform and calculating respective values of a haemodynamic characteristic in the corrected ultrasound waveform to determine the corrected values. Replacing the one or more irregular waveforms can mean removing the one or more irregular waveforms and using interpolation to determine the one or more corrected waveforms. Alternatively, replacing the one or more irregular waveforms can comprise removing the one or more irregular waveforms and replacing each irregular waveform with a template waveform.

In some embodiments, the method further comprises the steps of monitoring a number and/or duration of irregularities in the ultrasound waveform and determining whether one or more arrhythmia events have occurred based on the monitored number and/or duration of the irregularities. For example, an arrhythmia event can be determined to have occurred when there is a relatively large number of irregularities (within a particular period of time) and/or relatively long duration of irregularities.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer-implemented method of determining a measurement of a haemodynamic characteristic of a subject, the method comprising:

   obtaining (1001) an ultrasound waveform that comprises ultrasound data relating to a blood vessel of the subject, wherein the ultrasound waveform relates to a time period that includes a plurality of heartbeat cycles of the subject;
   processing (1003) the ultrasound waveform to detect a plurality of events in the ultrasound waveform, wherein the event is representative of a part of the heartbeat cycle;
   calculating (1005) respective values of a haemodynamic characteristic from the detected events;
   processing (1007) the calculated values of the haemodynamic characteristic to correct for irregularities in the ultrasound waveform and/or in the values of the haemodynamic characteristic, and to generate corrected values of the haemodynamic characteristic; and
   outputting (1009), via a display unit, the corrected values of the haemodynamic characteristic.

2. A method as claimed in claim 1, wherein the step of processing (1007) the calculated values comprises comparing the calculated values of the haemodynamic characteristic and smoothing a heartbeat-to-heart beat variation in the calculated values.

3. A method as claimed in claim 1 or 2, wherein the step of processing (1007) the calculated values comprises, for a first calculated value of the haemodynamic characteristic, generating a corrected value for the first calculated value as an average of the calculated values of the haemodynamic characteristic in a time window that includes the first calculated value.

4. A method as claimed in any of claims 1-3, wherein the step of processing (1007) the calculated values comprises:

   identifying one or more calculated values that result from a respective irregular part of the ultrasound waveform; and
   discarding the one or more identified calculated values, wherein the corrected values of the haemodynamic characteristic are the non-discarded calculated values.

5. A method as claimed in any of claims 1-3, wherein the step of processing (1007) the calculated values comprises:

   identifying one or more calculated values that result from a respective irregular part of the ultrasound waveform; and
   recalculating the values of the haemodynamic characteristic excluding the irregular parts of the ultrasound waveform associated with the one or more identified calculated values to calculate the corrected values.

6. A method as claimed in any of claims 1-3, wherein the step of processing (1007) the calculated values comprises:

   identifying one or more calculated values that result from a respective irregular part of the ultrasound waveform;
   replacing the one or more irregular parts in the ultrasound waveform with corrected waveforms to generate a corrected ultrasound waveform; and
   repeating the steps of processing the ultrasound waveform on the corrected ultrasound waveform and calculating respective values of a haemodynamic characteristic in the corrected ultrasound waveform to determine the corrected values.

7. A method as claimed in any of claims 1-6, wherein the method further comprises:

   monitoring a number and/or duration of irregularities in the ultrasound waveform; and
   determining whether one or more arrhythmia events have occurred based on the monitored number and/or duration of the irregularities.

8. A computer program product comprising a computer readable medium having computer readable code embodied therein, the computer readable code being configured such that, on execution by a suitable computer or processor, the computer or processor is caused to perform the method of any of claims 1-7.

9. An apparatus (2) configured to determine a measurement of a haemodynamic characteristic of a subject, the apparatus (2) comprises a processing unit (8) and a display unit (12), wherein the processing unit (8) is configured to:

   receive an ultrasound waveform that comprises ultrasound data relating to a blood vessel of the subject, wherein the ultrasound waveform relates to a time period that includes a plurality of heartbeat cycles of the subject;
   process the ultrasound waveform to detect a plurality of events in the ultrasound waveform, wherein the event is representative of a part of the heartbeat cycle;
   calculate respective values of a haemodynamic characteristic from the detected events;
   process the calculated values of the haemodynamic characteristic to correct for irregularities in the ultrasound waveform and/or in the values of the haemodynamic characteristic, and to generate corrected values of the haemodynamic characteristic; and
   output, via the display unit, the corrected values of the haemodynamic characteristic.

10. An apparatus (2) as claimed in claim 9, wherein the processing unit (8) is configured to process the calculated values by comparing the calculated values of the haemodynamic characteristic and smoothing a heartbeat-to-heart beat variation in the calculated values.

11. An apparatus (2) as claimed in claim 9 or 10, wherein the processing unit (8) is configured to process the calculated values by, for a first calculated value of the haemodynamic characteristic, generating a corrected value for the first calculated value as an average of the calculated values of the haemodynamic characteristic in a time window that includes the first calculated value.

12. An apparatus (2) as claimed in any of claims 9-11, wherein the processing unit (8) is configured to process the calculated values by:

identifying one or more calculated values that result from a respective irregular part of the ultrasound waveform; and

discarding the one or more identified calculated values, wherein the corrected values of the haemodynamic characteristic are the non-discarded calculated values.

13. An apparatus (2) as claimed in any of claims 9-11, wherein the processing unit (8) is configured to process the calculated values by:

identifying one or more calculated values that result from a respective irregular part of the ultrasound waveform; and

recalculating the values of the haemodynamic characteristic excluding the irregular parts of the ultrasound waveform associated with the one or more identified calculated values to calculate the corrected values.

14. An apparatus (2) as claimed in any of claims 9-11, wherein the processing unit (8) is configured to process the calculated values by:

identifying one or more calculated values that result from a respective irregular part of the ultrasound waveform; replacing the one or more irregular parts in the ultrasound waveform with corrected waveforms to generate a corrected ultrasound waveform; and

repeating the steps of processing the ultrasound waveform on the corrected ultrasound waveform and calculating respective values of a haemodynamic characteristic in the corrected ultrasound waveform to determine the corrected values.

15. An apparatus (2) as claimed in any of claims 9-14, wherein the processing unit (8) is further configured to:

monitor a number and/or duration of irregularities in the ultrasound waveform; and determine whether one or more arrhythmia events have occurred based on the monitored number and/or duration of the irregularities.

PWD Trace with HR Estimate Indexes

Fig. 1

Fig. 2

Fig. 3

EP 4 260 812 A1

Beat-to-Beat Common Carotid Stroke Volume (cSV CCA)

Fig. 4

Fig. 5A

EP 4 260 812 A1

EP 4 260 812 A1

Fig. 5B

Fig. 6

EP 4 260 812 A1

Fig. 7

EP 4 260 812 A1

Fig. 8

Fig. 9A

EP 4 260 812 A1

Fig. 9B

Fig. 9C

Fig. 9D

Obtain an ultrasound waveform that comprises ultrasound data relating to a blood vessel of the subject, the ultrasound waveform relating to a time period that includes a plurality of heart beat cycles of the subject ─1001

Process the ultrasound waveform to detect a plurality of events in the ultrasound waveform, with the events being representative of a part of the heart beat cycle ─1003

Calculate respective values of a haemodynamic characteristic from the detected events ─1005

Process the calculated values of the haemodynamic characteristic to correct for irregularities in the ultrasound waveform and/or values of the haemodynamic characteristic, and to generate corrected values of the haemodynamic characteristic ─1007

Output, via a display unit, the corrected values of the haemodynamic characteristic ─1009

Fig. 10

Europäisches
Patentamt

European
Patent Office

Office européen
des brevets

# EUROPEAN SEARCH REPORT

Application Number

EP 22 16 8339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/152592 A1 (HATIB FERAS [US] ET AL) 17 June 2010 (2010-06-17)<br>* paragraph [0019] *<br>* paragraph [0032] *<br>* paragraph [0034] *<br>* paragraph [0053] *<br>* paragraph [0018] *<br>* paragraph [0054] - paragraph [0055] *<br>* paragraph [0058] - paragraph [0059] *<br>* figure 3 * | 1-15 | INV.<br>A61B8/06<br>A61B5/029<br>A61B8/08 |
| X | WO 2011/094487 A2 (EDWARDS LIFESCIENCES CORP [US]; JIAN ZHONGPING [US]; HATIB FERAS [US]) 4 August 2011 (2011-08-04)<br>* pages 1, 2 *<br>* page 8, line 15 - page 20, line 22 * | 1,8,9 | |
| | | | TECHNICAL FIELDS SEARCHED (IPC)<br><br>A61B |

The present search report has been drawn up for all claims

| Place of search | Date of completion of the search | Examiner |
|---|---|---|
| Munich | 22 September 2022 | Ordavo, Ivan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 4 260 812 A1

### ANNEX TO THE EUROPEAN SEARCH REPORT
### ON EUROPEAN PATENT APPLICATION NO.

EP 22 16 8339

22-09-2022

| Patent document cited in search report | Publication date | Patent family member(s) | | Publication date |
|---|---|---|---|---|
| US 2010152592 A1 | 17-06-2010 | CA | 2689430 A1 | 19-02-2009 |
| | | CN | 101765398 A | 30-06-2010 |
| | | EP | 2175772 A2 | 21-04-2010 |
| | | US | 2009048527 A1 | 19-02-2009 |
| | | US | 2010152592 A1 | 17-06-2010 |
| | | WO | 2009023713 A2 | 19-02-2009 |
| WO 2011094487 A2 | 04-08-2011 | CN | 102834047 A | 19-12-2012 |
| | | EP | 2528499 A2 | 05-12-2012 |
| | | JP | 5850861 B2 | 03-02-2016 |
| | | JP | 2013517908 A | 20-05-2013 |
| | | US | 2013053664 A1 | 28-02-2013 |
| | | WO | 2011094487 A2 | 04-08-2011 |

EPO FORM P0459

For more details about this annex : see Official Journal of the European Patent Office, No. 12/82